# EUROPEAN PATENT APPLICATION

(11) **EP 1 544 212 A1**
(43) Date of publication of application: **22.06.2005**
(21) Application number: 03028623.1
(22) Date of filing: 15.12.2003
(51) Int. Cl.: C07K 14/765, A61K 38/38, A61P 31/12

(54) **Modified antiviral proteins with an increased negative charge**

(71) Applicant: ZLB Behring GmbH, 35041 Marburg (DE)
(72) Inventor: Friesen, Dr. Heinz-Jürgen, 35041 Marburg (DE)
(74) Representative: Pfeil, Hugo, Dr.

(57) **Abstract**

The invention relates to proteins and pharmaceutical preparations containing them which are suitable for treating or curing viral infections including orthomyxoviruses like influenza, paramyxoviruses like parainfluenza, measles and immunodeficiency diseases such as, but not limited to, retrovirus infections, including AIDS and AIDS-related diseases, and which also can be used to inhibit the fusion of virus-infected cells with non-infected cells.

## Description

The invention relates to proteins and pharmaceutical preparations containing them which are suitable for treating or curing viral infections including orthomyxoviruses like influenza, paramyxoviruses like parainfluenza, measles and immunodeficiency diseases such as, but not limited to, retrovirus infections, including AIDS and AIDS-related diseases, and which also can be used to inhibit the fusion of virus-infected cells with non-infected cells.

It is known from International patent application WO 92/15316 that pharmaceutical preparations are suitable for treating and curing of viral infections if they contain modified proteins or polypeptides as active substances which have acquired an additional net negative charge by derivatisation of their amino groups with a reagent which prevents protonation of their amino groups.

These results have been confirmed in US Patent 5.869.457 and it has been found for serum albumin and other proteins that a correlation exists between the negative charge of the modified protein and its antiviral activity and more specifically between the introduced mean negative charge per amino acid and the increase in antiviral activity of the protein (P.J. Swart et al, J.Peptide Sci. 5: 563-576, 1999). The more negative the protein is charged by a suitable chemical modification, the greater is its antiviral activity, in particular its anti-HIV activity.

In addition several scientific publications have shown that negatively charged proteins are potent inhibitors of HIV-1 replication in vitro and that succinylated and acotinylated proteins act at an early stage of the virus life cycle (Jansen, R.W., *et al.* (1991) Mol Pharmacol, 39; 818-823, Jansen, R.W., *et al.* (1993) Mol Pharmacol, 44; 1003-1007). It has been confirmed that by a direct interaction of negatively charged proteins with HIV-1 gp120 the binding of the virus through gp120 with CD4-cells is blocked.

The term "protein or polypeptide" in the sense of the invention presented here denotes any natural protein or a fragment (see below), or a variant of the protein or polypeptide. Preferred are mammalian proteins, more preferred are human proteins for human applications and here most preferred are plasma proteins like albumin, immunoglobulins, transferrin and fibrinogen. The scope of the invention is illustrated in more details with albumin, its fragments and variants; but is not limited to albumin.

It is now suggested to achieve the antiviral effect of the protein by preparing sequence variants in such a way that the basic amino acids, such as lysine, arginine and histidine, preferably lysine, which carry positive charges in the side chain, are replaced by amino acids carrying negatively charged side chains, like aspartic acid or glutamic acid, by recombinant DNA technology. This invention also includes the possibility to replace the basic amino acids in the native protein by other amino acids containing negative charges in the side chain, like cysteic acid or homocysteic acid or O-phosphoserin. The spacer between the alpha carbon atom of such an amino acid and the side chain acidic function can be a number of methylene groups or a number of more polar groups like ethoxy groups or carbohydrate structures like in dextranes or heparins. Incorporation of other amino acids than those introduced using the coding for the 21 known natural amino acids can be achieved by manipulating the genetic code accordingly.

For such protein sequence variants the term "modified protein or polypeptide" is also used in the context of this invention. Unmodified proteins or polypeptides are also referred to as "wild-type" proteins or polypeptides.

Preferably, the number of basic amino acids replaced by acidic amino acids is chosen such that a minimum charge difference per amino acid compared to the parent protein sequence of more than 0.1 and most preferably more than 0.15 results. This can also be measured by the prolongation of the retention time of the protein sequence variant according to the invention, compared with that of the parent protein, the retention time being determined by means of a FPLC system (Amersham-Pharmacia) on an anion exchange column, as described by Jansen et al., Mol. Pharmacology 39: 818-823, 1991 and Mol. Pharmacology 44: 1003-1007, 1993.

As little as the exchange of one basic amino-acid can have an effect, a more pronounced effect will occur if 10, 20, 30, 40, 50, 60, 70, 80, 90 or up to 100 % of the basic amino acids are exchanged.

It is now further suggested to increase the antiviral effect of a negatively charged protein in the sense of this invention by fusion with a protein or polypeptide selected from the class of the "entry inhibitors". Said protein or fragment fused or contemplated to be fused with an entry inhibitor (see fusion protein below) has at least one of its positively charged groups chemically modified into a negative moiety. Preferably, the number of basic amino acids replaced by acidic amino acids is chosen such that a minimum charge difference per amino acid compared to the parent protein sequence of more than 0.1 and most preferably more than 0.15 results.

A "fragment", in the context of albumin (or any other protein within the meaning of this application, see e.g. P. J. Swart et al., J. Peptide Sci. 5:563-576, 1999; suitable proteins are immunoglobulins or milk proteins as lactoglobulin or lactoferrin. In case of lactoferrin as a fusion partner which by itself has antiviral activities a "trifusion" construct of e.g. albumin - lactoferrin - (entry-inhibitor protein) is preferred), refers to a protein wherein at least one position therein has been deleted. Thus the fragment may comprise at least 5, 10, 20, 30, 40 or 50 % of the complete sequence of mature protein. Typically a fragment comprises up to 60 %, more typically up to 70 %, preferably up to 80 %, more preferably up to 90 %, even more preferably up to 95 %, yet more preferably up to 99 % of the complete sequence of albumin. Particularly preferred fragments of the proteins comprise one or more whole domains of the protein. Albumin has three domains. A particularly preferred fragment of albumin may contain one or two domains and will thus typically comprise at least 33 % or at least 66 % of the complete sequence of albumin. Domain 1 consists of amino acids 1-194 of mature human serum albumin, domain 2 is amino acids 195-387 and domain 3 is amino acids 388-585.

The term "albumin" is used herein to denote material, which is indistinguishable from serum albumin or which is a variant or fragment thereof. By "variant" we include insertions, deletions and substitutions, either conservative or non-conservative, where such changes do not substantially alter the oncotic, useful ligand-binding or immunogenic properties of albumin. For example we include naturally-occurring polymorphic variants of albumin or albumin analogues disclosed in EP-A-322 094. Generally, variants or fragments of albumin will have at least 10 % (preferably at least 50 %, 80 %, 90 % or 95 %) of serum albumin's ligand binding activity (for example bilirubin-binding) and at least 50% (preferably at least 80 %, 90 % or 95 %) of human serum albumin's oncotic activity, weight for weight. Oncotic activity, also known as colloid osmotic pressure, of albumin, albumin variants or fragments of albumin may be determined by the method described by Hoefs, J.C. (1992) *Hepatology* **16**:396-403. Bilirubin binding may be measured by fluorescence enhancement at 527 nm relative to HSA. Bilirubin (1.0mg) is dissolved in 50µL of 1M NaOH and diluted to 1.0mL with demineralised water. The bilirubin stock is diluted in 100mM Tris-HCI pH8.5,1mM EDTA to give 0.6nmol of bilirubin mL⁻¹ in a fluorometer cuvette. Fluorescence is measured by excitation at 448nm and emission at 527nm (10nm slit widths) during titration with HSA over a range of HSA:bilirubin ratios from 0 to 5 mol:mol.

The term "fusion" is used herein to denote a covalent or non-covalent linkage between the albumin and the entry inhibitor protein or polypeptide. Those skilled in the art will recognise that there are a number of alternative mechanisms to achieve such a linkage. When the linkage is non-covalent, the basic amino acids can be replaced by acidic amino acids before linkage to the entry inhibitor protein or polypeptide. When the linkage is covalent, the basic amino acids of the albumin can be replaced by acidic amino acids before linkage to the entry inhibitor protein or polypeptide if, for instance, bi-functional cross-linkers are employed. When fusion is achieved by genetically fusing the DNA sequences encoding the albumin and the entry inhibitor protein or polypeptide basic amino acids in the fusion inhibitor part or the albumin part or both may be replaced by acidic amino acids.

In cases where the replacement of basic amino acids in the entry inhibitor as part of such a fusion protein is deleterious to its desired activity, these positions in the sequence may be left untouched or a replacement doing no harm to the entry inhibitor's activity may be chosen.

Typically when the bond is covalent, bi-functional cross-linkers such as 1,4-bis-maleimidobutane (BMB), 1,4-bis-maleimidyl-2,3-dihydroxybutane (BMDB), bis-maleimidohexane (BMH), bis-maleimidoethane (BMOE), 1,8-bis-maleimidotriethyleneglycol (BM[POE]₃), 1,11-bis-maleimidotetra-ethyleneglycol (BM[POE]₄), 1,4-di[3N-(2N-pyridyldithio)-propionamido]-butane (DPDPB), dithio-bis-maleimidoethane (DTME) or 1 ,6-hexane-bis-vinylsulfone (HBVS) can be used to join the albumin to the "entry inhibitor" protein or polypeptide. Also heterobifunctional crosslinkers carrying for example an amino group- specific reactive function, like an active ester, at one end and a sulfhydril- specific reactive function, like a maleimido-group, at the other end may be used. With higher molecular weight spacers, like polyethylene glycols, benefical effects from the nature of the spacer, like improved sterical conditions, overall increase in molecular weight resulting in improved halflife time or reduced immunogenicity can result.

Alternatively, the "entry inhibitor" protein or polypeptide may be modified by fusion with an albumin ligand which will typically bind to albumin, which has basic amino acids replaced by acidic amino acids, non-covalently. Any appropriate ligand may be used, provided that it specifically binds to such albumins. The albumin ligand may, for example, comprise the sequence of an antibody. Thus, for example, it may be an IgG molecule. In this context, the term antibody also includes fragments and portions thereof that retain sequences involved in binding. The variable heavy (V_{H}) and variable light (V_{L}) domains of the antibody are involved in antigen recognition and binding, a fact first recognised by early protease digestion experiments. Further confirmation was found by "humanisation" of rodent antibodies.

Thus, a fragment may contain one or more of the variable heavy (V_{H}) or variable light (V_{L}) domains. For example, the term antibody fragment includes Fab-like molecules (Better *et al* (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward *et al* (1989) Nature 341, 544).

Where the variable domain(s) used are not derived from the same species as the individual intended for treatment, the antibody may be modified by fusion of those variable domains(s) to constant domains derived from the species of individual intended for treatment. Thus, where a human is intended for treatment, the antibody may be "humanised" by fusion of the variable domain(s) to constant domains of human origin such that the resultant antibody retains the antigenic specificity of the parent antibody (for example, see Morrison *et al* (1984) Proc. Natl. Acad. Sci. USA 81, 6851-6855).

A general review of the techniques involved in the synthesis of antibody fragments which retain their specific binding sites is to be found in Winter & Milstein (1991) Nature 349, 293-299.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted recombinantly (e.g. from E.coli or yeast such as *Saccharomyces cerevisae*), thus allowing the facile production of large amounts of the said fragments.

The skilled person will be aware of alternatives to using antibodies. For example, the albumin ligand may comprise the amino acid sequence DICLPRWGCLW, a peptide sequence known to bind to albumin with high specificity (Dennis *et al,* 2002, *J. Biol. Chem.,* **277**: 35035), or a variant thereof, which substantially retains the same albumin-binding properties.

Entry inhibitors are agents that prevent viruses from infecting target cells, and within this class, at least three subclasses already exist. The penetration of cells by viruses like HIV is a multi-step process. At first HIV must recognize and bind to two separate molecules on the outer membrane of the target cell, CD4- and chemokine-receptors (CCR5 and/or CXCR4). Once the virus attaches itself to the cell surface, fusion occurs, the viral and cell membranes merge and the viral core is inserted into the cell cytoplasm. Each of these three events, CD4-receptor binding or attachment, chemokine- or co-receptor binding and fusion is an independent target for drug intervention.

The infection of a non-infected cell by an infected cell involves essentially the same steps.

Entry inhibitors to which a larger molecule like albumin has been attached exhibit a beneficial prolongated half-life time. This has been described e.g. in WO 01/79444.

Subject of the present invention are therefore proteins, which are found in nature, their fragments or variants in which basic amino acids have been replaced by acidic amino acids to achieve or enhance an anti-viral effect.

Subject of the present invention are therefore also new antiviral proteins which are prepared by fusion of proteins with a protein or polypeptide selected from the group of entry inhibitors which are capable of inhibiting the fusion of virus-infected cells with non-infected cells, wherein basic amino acids have been replaced by acidic amino acids.

Examples of such entry inhibitors, which are polypeptides or proteins by nature, are CD4, T-20, T-1249, 5-Helix, DP-178 or cyanovirin. The scientific background of the similarities in the mechanism of the fusion steps of retroviruses like HIV and SIV and paramyxoviruses like measles are outlined in e.g. Lambert et al., PNAS 93, 2186-2191, 1996. Orthomyxovirus, i.e. influenza, replication is also inhibited by modified proteins. Entry inhibitors for HIV, SIV and measles are described e.g. in US Patents 6.020.459, 6.017.536, and 6.013.263, respectively. A compilation of entry inhibitors presently in development is disclosed in detail in the Datamonitor Report of February 2001 entitled "Entry Inhibitors in HIV" (www.datamonitor.com).

Albumin fusion products are described in International patent applications WO 9724445, 0179480, 0179442, 0179443, 0179444, 0179271 and 0179258 and US Patent 6.165.470. Such an albumin fusion product may be obtained by the coupling, through chemical or genetic engineering techniques, of at least two distinct functions, namely a stable plasma transporter function provided by human serum albumin (HSA) and an entry inhibitor function, which prevents the invasion of the virus into the non-infected cell or the fusion of a virus infected cell with a non-infected cell. Such coupling processes are shown in US Patent 6.165.470 for the hybrid macromolecule comprising HSA-CD4 and for various other albumin fusions in International patent applications WO 9724445, 0179480, 0179442, 0179443, 0179444, 0179271 and 0179258, but can also be performed for the coupling of HSA with the entry inhibitors of polypeptide or protein nature like those mentioned above. All these hybrid macromolecules exhibit a biological activity against the fusion of virus or virus-infected cells with non-infected cells.

The antiviral potency of these compounds however can be considerably increased if such an albumin fusion protein containing a fusion inhibitor is generated by replacing basic amino acids with acidic amino acids as described above.

Known entry inhibitors act at different sites and thus exhibit variations of the basic mechanism of the fusion and entry inhibitor effects.

Cyanovirin-N preferably targets N-linked, high mannose glycans in an oligosaccharide-specific manner on e.g. gp120 in HIV or such structures in herpes viruses (Shenoy SR et al. 2001. Selective interactions of the HIV-inactivating protein, cyanovirin-N, targets high-mannose oligosaccharides on gp120 and other glycoproteins. J. Pharmacol. Exp. Ther. 2972-704-710; Bolmstedt AJ *et al.* 2001. Cyanovirin-N defines a new class of antiviral agent targeting N-linked, high mannose glycans in an oligosaccharide-specific manner. Mol. Pharmacol. 5905: 949-954).

T-20 and T-1249 target the fusion process between viral and cellular membrane during virus entry into the cell. They are small molecules that bind to crucial helical structures in transition states involved in the fusion process thus providing an effective method of inhibiting the virus (for reviews see Chan DC and Kim PS. 1998. HIV Entry and its Inhibition, Cell 93:681-684; Doranz BJ 2000. The use of Envelope for HIV therapeutics: from Vaccines to Co-receptors. Emerging Therapeutic Targets 4:423-437; LaBranche CC 2001. HIV fusion and its inhibition. Antiviral Research 50:95-115) (Wild CT *et al.* 1994. Peptides corresponding to a predictive alphahelical domain of human immunodeficiency virus type 1 gp41 are potent inhibitors of virus infection. Proc. Natl. Acad. Sci. 91:9770-9774).

The mechanism of the action of 5-Helix was described in 2001 (Root MJ 2001. Protein design of an HIV-1 entry inhibitor. Science 291:884-888). The binding of 5-Helix is thought to prevent the conformational changes on gp41 associated with the fusion event differently from T-20 or T-1249 and thereby also prevent infection of the cell.

The entry inhibitor and the modified protein parts of these novel protein/entry inhibitor fusion products can interfere at different sites and times during the virus-cell-fusion step. Especially binding of these two parts at different sites with suitable proximity is beneficial as it improves both the binding kinetics and the binding strength. On the basis of this double-action mechanism, the entry inhibitor and the modified protein part show more than additive effects and improvements over these parts taken alone, i.e. a synergistic action. Another benefit of this concept takes action in cases where the virus evades the mechanism of one of the two parts of the two fused antiviral partners by mutagenesis. The fusion of entry inhibitor and a larger protein is a tool to provide enhanced half-life time compared especially to an entry inhibitor of lower molecular weight.

The modified proteins which contain additional net negative charges can be used in the pharmaceutical preparations according to the invention. Compounds of this type are generally water-soluble and they can exist in ionic form and have a molecular weight of about 50,000 Da. However, the molecular weight can also be appreciably higher or lower, the important factor is that a poly-anionic polypeptide structure is present in the molecule.

Medicinal preparations which contain the negatively charged antiviral protein according to the invention can also be in the form of powders, suspensions, solutions, sprays, emulsions, ointments or creams and can be used for topical application, for intranasal, rectal or vaginal administration, and also for oral or parenteral (intravenous, intradermal, intramuscular, intrathecal, and the like) administration. Such preparations can be prepared by combining the negatively charged antiviral protein (for example by mixing, dissolving or the like) with pharmaceutically acceptable excipients of a neutral type, such as aqueous or non-aqueous solvents, stabilizers, emulsifiers, detergents and additives, and also, if desired, with colorants and aroma substances. The concentration of the negatively charged antiviral protein in the preparation according to the invention can vary substantially and be between 0,001, preferably 0,1 and 100 %, depending on the mode of administration. Furthermore, the dose of the negatively charged antiviral protein to be administered can, for example, be between 0,1 mg and 100 mg/kg of body weight.

It is expected that by intravenous administration of the fused antiviral protein as described according to the present invention either by infusions or by repeated single injection usually with a total input per day of a least 1,5 mg/kg body weight and maximally 15 mg/kg, long lasting antiviral concentrations will be attained in the blood stream and the lymphatic system of virus infected patients.

The invention also relates to the use of the antiviral protein according to the invention which has acquired additional net negative charges, as defined above, as active substance or as substance contributing to the action, or as carrier for active substance when preparing pharmaceutical preparations and immunodeficiency diseases such as retrovirus infections, including AIDS and AIDS-related diseases, and which can be used to inhibit fusion of infected with non-infected cells.

The invention also relates to a method for treating diseases or disorders caused by retrovirus infections such as AIDS and AIDS-related diseases, or conditions in which fusion of virus with cells or fusion of virus-infected cells with non-infected cells occurs, with which method an additionally negatively charged protein as defined above is used.

The invention also relates to a method for the preparation of antiviral protein by replacing basic amino acids by acidic amino acids, the proteins or polypeptides acquiring additional net negative charges such, that a minimum average charge difference per amino acid of more than 0.1 and most preferably more than 0.15 results compared to the parent protein or polypeptide. The charge difference results from exchanging basic amino acids, which carry one positive charge at neutral pH in their side chain, with acidic amino acids, which carry one negative (e.g. aspartic acid or glutamic acid result in a charge difference of -2 per amino acid exchange) or more negative charges (e.g. phosphoserin results in a charge difference of -3 per amino acid exchange) in their side chain. The average charge difference per amino acid results from the sum of the charge differences induced by the amino acid exchanges divided by the total number of amino acids in the respective sequence as described in P.J. Swart et al, J.Peptide Sci. 5: 563-576, 1999. The effect of the exchange of basic amino acids by acidic amino acids can also be measured by the prolongation of the retention time of the antiviral protein according to the invention, compared with that of the parent protein, the retention time being determined by means of a FPLC system (Amersham-Pharmacia) on an anion exchange column, as described by Jansen et al., Mol. Pharmacology 39: 818-823, 1991 and Mol. Pharmacology 44: 1003-1007, 1993.

According to the invention, it is suggested that the negatively charged antiviral protein is, inter alia, a powerful and selective anti-HIV agent which substantially reduces syncytium formation and virus-cell fusion, does not bind to the OKT4A epitope of the CD4 receptor and has only a slight inhibitory action on the interaction of anti-gp120 and mAb gp120 and the binding of virus cells at very low concentrations. The negatively charged antiviral protein used or prepared according to the invention has no or only a low toxicity.

The invention is illustrated with reference to the following examples:

### 1) Substitution of lysine residues by aspartic or glutamic acid residues

Yeast expression vectors and methods for recombinant human albumin expression have been described in detail in Sleep D *et al.* (1991) BioTechnology 9:183-187 and in patent applications WO 00/044772 and WO 03/066078. For site-directed substitution of lysine residues one skilled in the art would use a commercially available mutagenesis kit such as the QuikChange® XL Site-Directed Mutagenesis Kit available from Stratagene (Catalog No. 200517). Primers replacing the AAA and AAG codons encoding lysine by either GAT or GAC encoding aspartic acid or GAA or GAG encoding glutamic acid are being designed according to the kit protocol. Seq-ID No. 1 illustrates the human albumin sequence and the lysine residues amenable to substitution. The substitutions can be carried out either in one, two or all three domains of the albumin molecule. The degree of substitution is guided by the rule, that the average charge difference introduced by the site-directed substitutions is at least 0.1 or preferably more than 0.15 per amino acid in the respective protein sequence.

### 2) Yeast transformation and culturing conditions

Yeast strains disclosed in WO 95/23857, WO 95/33833 and WO 94/04687 are transformed to leucine prototrophy as described in Sleep D., *et al.* (2001) Yeast 18:403-421. The transformants are patched out onto Buffered Minimal Medium (BMM, described by Kerry-Williams, S.M. *et al.* (1998) Yeast 14:161-169) and incubated at 30°C until grown sufficiently for further analysis.

### 3) Expression and purification of modified human albumin

Recombinant modified human albumin is expressed in shake flask culture and the expression levels are measured by SDS-PAGE using an albumin standard.

### 4) Purification of modified human albumin

The antiviral protein comprising an albumin sequence variant with basic amino acids replaced by acidic amino acids is purified using
a) a cation exchange chromatography such as SP-FF and b) an anion exchange chromatography such as DE-FF , both purification steps with pH and salt adjusted to the specific charge properties of this antiviral protein to achieve adequate purification and keep the components in solution, as is known to a person skilled in the art. The purified material is then concentrated and diafiltered against a suitable buffer, e.g. PBS. The pore size of the membrane is chosen such that the antiviral protein is retained in the retentate.

Depending on the properties of the antiviral protein and the desired degree of purification it can be advantageous to include further purification steps and additional purification principles like hydrophobic interaction chromatography, metal chelate affinity chromatography or affinity chromatography involving antibodies specifically binding to the antiviral protein, in the purification scheme.

Seq ID No. 1, human serum albumin. This amino acid sequence represents the primary translation product and, therefore, includes a 24 amino acid leader sequence which is removed during secretion in yeast. Lysine residues amenable for substitution are underlined and numbered.

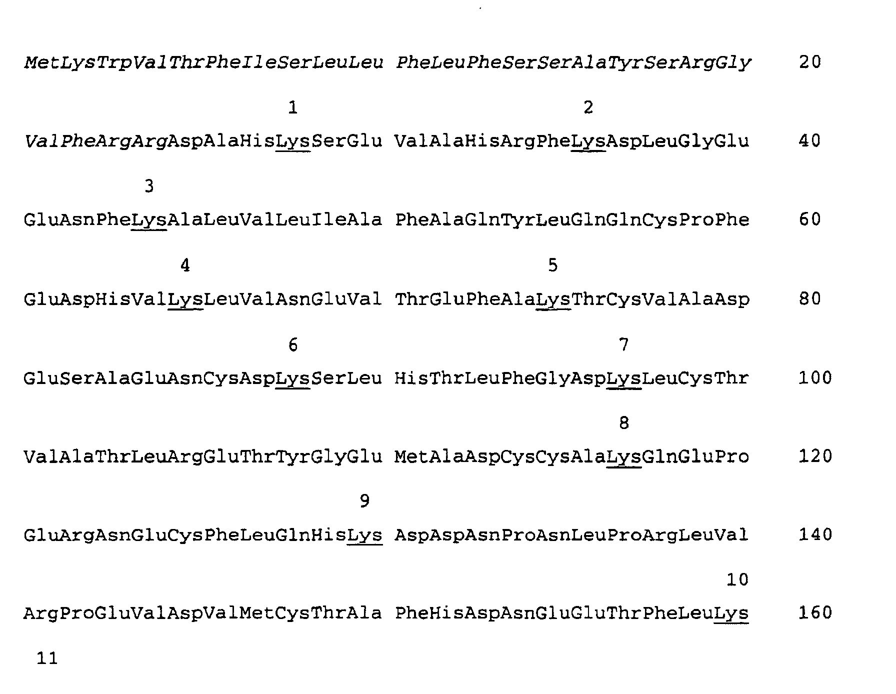

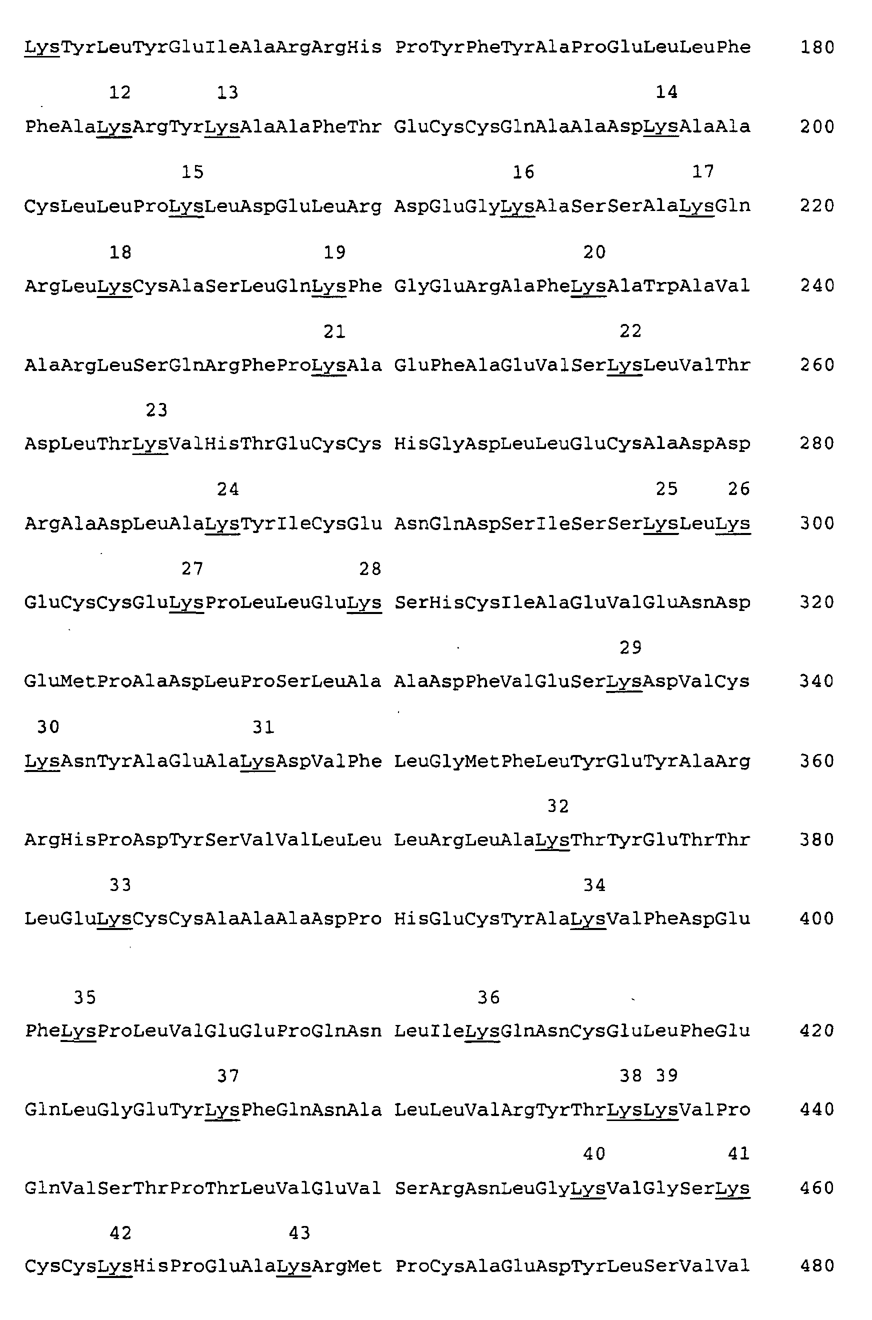

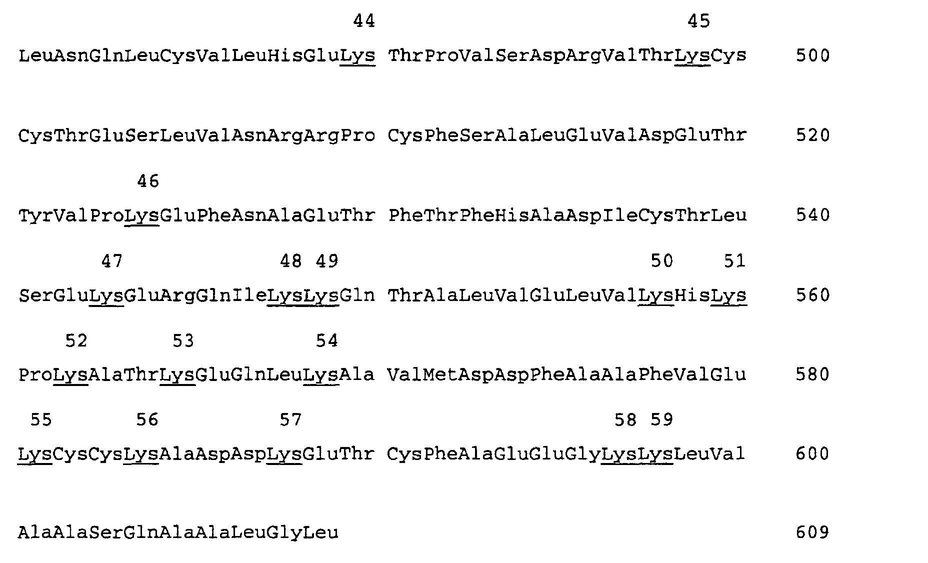

### 5) Preparation of negatively charged, antiviral albumin fusion proteins according to the invention

A T20-fusion macromolecule sequence prepared in accordance with U.S. patent 6.165.470 is modified in such a way that lysines in the native albumin sequence are replaced by amino acids carrying negative charges in the side chains by recombinant methodology known to the expert scientist. As a result the T20- albumin molecules acquires additional net negative charges compared to the native albumin sequence. This derivatisation has the effect that the retention time of the T20- albumin molecule on an anion exchange column is prolonged compared with the retention time of the molecule containing the native albumin sequence, the retention time being determined by means of a FPLC system (=Fast Protein Liquid Chromatography) from Amersham-Pharmacia on an anion exchange column as described by Jansen et al., Mol. Pharmacology 44: 1003-1007, 1993.

### 6) Anti-HIV-1-Test in the presence of an antiviral albumin according to the invention

A Pharmacia Mono-Q HR 5/5 equilibrated with 20 mM TRIS-HCI buffer, pH 7.5, is run at 0.25 ml/min. The eluate is monitored at 280 nm. A sample, 0.1 ml, is injected onto the column and the column run with a 30 minute linear gradient to 20 mM TRIS-HCl, pH 7.5, containing 1 M NaCl.
The retention times of the albumin sequence variant compared to the molecules with the native albumin sequence are prolonged.

### 7) Anti-HIV-1-Test in the presence of an antiviral albumin according to the invention

Jurkat cells (e.g. 5x10E5/ml) are mixed with HIV inhibitor or control protein solution and HIV-1 (e.g. 1.5x10E5 CCID50/ml), each sample being assayed in 8 replicates in 98-well plates, and incubated at 37°C for 4 days. Each sample is evaluated for the amount of cell-cell fusions, antiviral activity being measured through reduction of fusion events compared to control.

SEQUENCE LISTING

<110>  AVENTIS BEHRING GMBH

       AVENTIS BEHRING GMBH

<120>  Antiviral Protein Sequence Variants

<130>  2003/M020-A82

<140>  03028623.1

<141>  2003-12-15

<160>  1

<170>  PatentIn version 3.1

<210>  1

<211>  609

<212>  PRT

<213>  Homo sapiens

<400>  1

## Claims

1. Modified protein or polypeptide comprising a protein or polypeptide in which at least one basic amino acid of the wild-type protein or polypeptide has been replaced by an amino acid carrying a negative charge in their side chain.

2. Modified protein or polypeptide according to claim 1, which is capable of inhibiting the fusion of virus and/or virus-infected cells with non-infected cells.

3. Modified protein or polypeptide according to claim 1 or 2, wherein such a number of basic amino acids of the wild-type protein or polypeptide has been replaced by amino acids carrying a negative charge in their side chain, that a minimum average charge difference per amino acid of more than 0.1 and most preferably more than 0.15 results.

4. Modified protein or polypeptide according to any of claims 1 to 3, wherein the basic amino acids are chosen from the group of lysines, arginines or histidines.

5. Modified protein or polypeptide according to any of claims 1 to 4, wherein the protein sequence of the wild-type protein or polypeptide is a sequence fragment or a variant of a wild-type protein.

6. Modified protein or polypeptide according to any of claims 1 to 5 wherein the wild-type protein is a mammalian protein.

7. Modified protein or polypeptide according to claim 6 wherein the mammalian protein is a human protein.

8. Modified protein or polypeptide according to claim 7 wherein the human protein is a plasma protein.

9. Modified protein or polypeptide according to claim 8 wherein the human protein is human albumin.

10. Modified protein or polypeptide according to any of claims 1 to 9, wherein a second protein or polypeptide belonging to the class of viral fusion inhibitors is fused to modified protein or polypeptide.

11. Modified protein or polypeptide according to claim 10, wherein the second anti-viral protein or polypeptide is a modified protein or polypeptide according to claim 3.

12. Pharmaceutical preparation for treating or curing viral infections comprising a modified protein or polypeptide according to any of claims 1 to 11 as an active substance or as a substance contributing to the action or as a carrier for other substances, which are capable of inhibiting the fusion of virus-infected cells with non- infected cells.

13. Use of a modified protein or polypeptide according to any of claims 1 to 11 in a method of treating viral diseases or disorders caused by a virus or retrovirus infection

14. Process for the preparation of an antiviral protein as claimed in claims 1 to 12, wherein the basic amino acids of a protein are replaced by acidic amino acids such, that a minimum average charge difference per amino acid of more than 0.1 and most preferably more than 0.15 results.

15. Use of a modified protein or polypeptide according to any of claims 1 to 11 for the preparation of a medicament for the treatment of viral infections.

16. Use according to claim 15, wherein the viral infection is an HIV-infection.
